# EUROPEAN PATENT APPLICATION

(11) **EP 4 813 282 A1**
(43) Date of publication of application: **30.09.2026**
(21) Application number: 26166733.1
(22) Date of filing: 23.03.2026
(51) Int. Cl.: A61B 1/00, A61B 1/055, A61B 1/313

(54) **MEDICAL IMAGE PROCESSING DEVICE AND METHOD OF OPERATING THE SAME**

(30) Priority: 25.03.2025 JP 2025049264
(71) Applicant: FUJIFILM Corporation, Tokyo 106-8620 (JP)
(72) Inventor: KITAHARA, Masahiro, Tokyo (JP)
(74) Representative: Dehns Germany Partnerschaft mbB

(57) **Abstract**

Provided are a medical image processing device (32) that reduces a burden on a user such as a doctor or a nurse during calibration, and a method of operating the same.

An image acquisition unit (34) acquires an image. A mode controller (35) switches to a calibration mode in a case where the mode controller (35) recognizes, from the image, a pattern (20, 22) used for correcting the image. The mode controller (35) switches from the calibration mode to a normal mode in a case where a release condition related to the correction is satisfied. The release condition is that an evaluation of the correction is appropriate.

## Description

### BACKGROUND OF THE INVENTION

### 1. Field of the Invention

The present disclosure relates to a medical image processing device that performs calibration before insertion into a body, and a method of operating the same.

### 2. Description of the Related Art

In the medical field, an endoscope is used to observe an inside of a patient's body. Since the endoscope has individual differences in an imaging optical system used for imaging inside the body, for example, as disclosed in JP2024-52487A, calibration is performed before observation to correct differences in colors or the like on an image caused by individual differences of the endoscope.

### SUMMARY OF THE INVENTION

As the endoscope, in addition to an endoscope for observing a digestive tract or the like, a rigid endoscope for observing an abdominal cavity is also used. In recent years, an application for improving diagnosis has been implemented using an image obtained by the rigid endoscope. For example, an ultrasound probe is inserted into the abdominal cavity, and an ultrasound image obtained from the ultrasound probe is two-dimensionally projected onto an image obtained by the rigid endoscope and displayed in a superimposed manner. In addition, an orientation of an organ is detected from the image obtained by the rigid endoscope, and a three-dimensional model such as a preoperative 3D is reconstructed based on the detected orientation.

In a case of attempting to implement the application as described above, the image obtained by the rigid endoscope is highly distorted due to a wide field of view of the rigid endoscope, so that misregistration occurs in a case where the image obtained by the rigid endoscope is superimposed on the ultrasound image. In addition, the orientation of the organ cannot be correctly detected from the image obtained by the rigid endoscope, and it may be impossible to correctly reconstruct the three-dimensional model. To address this issue, as in JP2024-52487A, by imaging a calibration pattern in advance using a normal computer vision task and obtaining parameters for correcting image distortion, a problem such as misregistration due to image distortion can be avoided.

On the other hand, since the rigid endoscope is mounted on a camera head and is not electrically connected to a system that processes the image, in a case where a doctor or a nurse changes the rigid endoscope during a surgery as necessary, the doctor or the nurse needs to perform calibration at the time of change. The calibration is performed through a series of operations of 1) switching the system to a calibration mode, 2) placing the rigid endoscope and the calibration pattern at appropriate positions, and 3) performing imaging.

In a case of performing the above series of operations, it is difficult to operate an operation button of the system while holding the rigid endoscope and the calibration pattern with both hands. Therefore, it is necessary to perform the calibration with two or more workers or to secure a place for installing the calibration pattern in an operating room.

An object of the present disclosure is to provide a medical image processing device that reduces a burden on a user such as a doctor or a nurse during calibration, and a method of operating the same.

According to an aspect of the present disclosure, there is provided a medical image processing device comprising: a processor, in which the processor is configured to acquire an image, and switch to a calibration mode in a case where a pattern used for correcting the image is recognized from the image.

It is preferable that the processor be configured to switch from the calibration mode to a normal mode in a case where a release condition related to the correction is satisfied, and maintain the calibration mode in a case where the release condition is not satisfied. It is preferable that the release condition be that an evaluation of the correction is appropriate.

It is preferable that the processor be configured to estimate a parameter of the correction from feature points of the image. It is preferable that the processor be configured to estimate the parameter from a plurality of the images in which the pattern has different orientations. It is preferable that the processor be configured to evaluate the correction based on the image reflecting the parameter. It is preferable that the processor be configured to store the parameter in a case where the evaluation is appropriate.

It is preferable that the processor be configured to display a guide pattern for guiding the pattern on the image in the calibration mode. It is preferable that the processor be configured to change the display of the guide pattern in accordance with an orientation of the pattern that is deemed appropriate as an orientation for performing the correction.

It is preferable that the processor be configured to determine whether or not an orientation of the pattern on the image is appropriate as an orientation for performing the correction, and in a case where the orientation of the pattern is appropriate, notify that the orientation of the pattern is appropriate.

According to another aspect of the present disclosure, there is provided a method of operating a medical image processing device including a processor, the method comprising: a step of acquiring an image via the processor; and a step of switching to a calibration mode in a case where the processor recognizes, from the image, a pattern used for correcting the image.

According to the present disclosure, it is possible to reduce a burden on a user such as a doctor or a nurse during calibration.

### BRIEF DESCRIPTION OF THE DRAWINGS

FIG. 1 is a schematic diagram showing a laparoscopic surgery.
FIG. 2 is an explanatory diagram showing a method of imaging a calibration plate.
FIG. 3 is a plan view of a calibration plate on which a pattern is provided.
FIG. 4 is a block diagram showing functions of the endoscope system.
FIG. 5 is an image diagram of an image on which a pattern is displayed.
FIG. 6 is an image diagram of an image on which a guide pattern is superimposed.
FIG. 7 is an explanatory diagram showing determination of an orientation of a pattern in a sequence of video images.
FIG. 8 is an explanatory diagram showing that an orientation of a pattern is appropriate by changing an outer frame of an image.
(A) of FIG. 9 is an explanatory diagram showing a guide pattern for a first orientation, and (B) of FIG. 9 is an explanatory diagram showing a case where the guide pattern for the first orientation overlaps a pattern.
(A) of FIG. 10 is an explanatory diagram showing a guide pattern for a second orientation, and (B) of FIG. 10 is an explanatory diagram showing a case where the guide pattern for the second orientation overlaps a pattern.

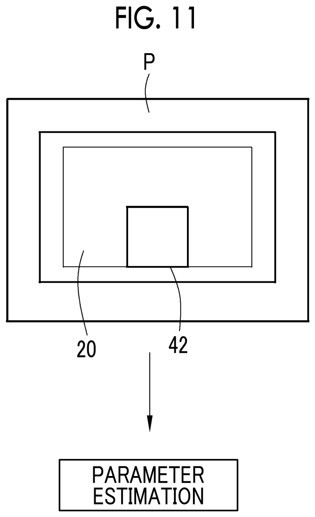

FIG. 11 is an explanatory diagram showing estimation of a parameter.

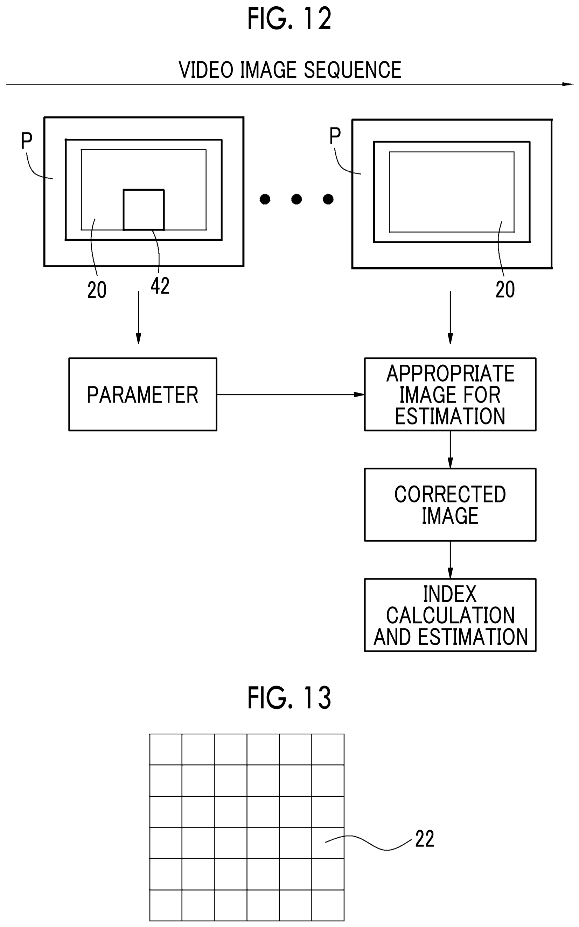

FIG. 12 is an explanatory diagram showing a corrected image reflecting a parameter, calculation of an index value from the corrected image, and evaluation of the parameter.
FIG. 13 is an explanatory diagram showing a pattern for evaluation.

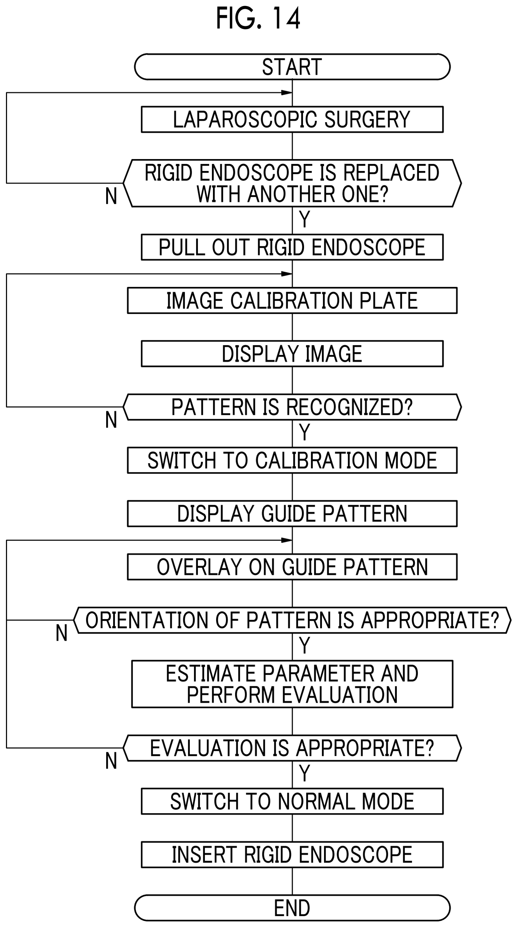

FIG. 14 is a flowchart showing a series of flows of calibration.

### DESCRIPTION OF THE PREFERRED EMBODIMENTS

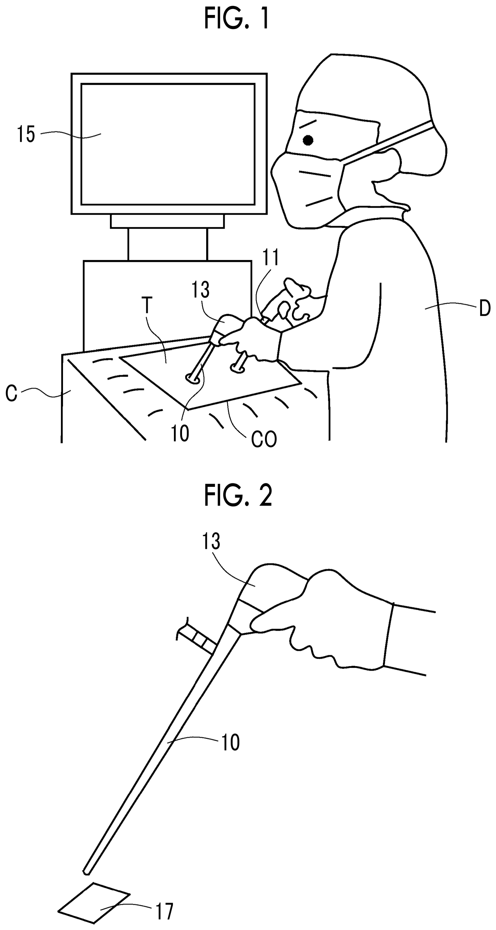

As shown in FIG. 1, in a laparoscopic surgery, a cover C that covers a patient is provided with an opening portion CO, and a rigid endoscope 10 and forceps 11 are inserted into a surgical target part T of the patient, which is exposed through the opening portion CO. During the surgery, a doctor D holds a camera head 13 to which the rigid endoscope 10 is connected and the forceps 11. An image of a patient's abdominal cavity obtained from the camera head 13 is displayed on a display 15. The doctor D performs the laparoscopic surgery by operating the camera head 13 or the forceps 11 that is held by the doctor D while observing the image on the display 15.

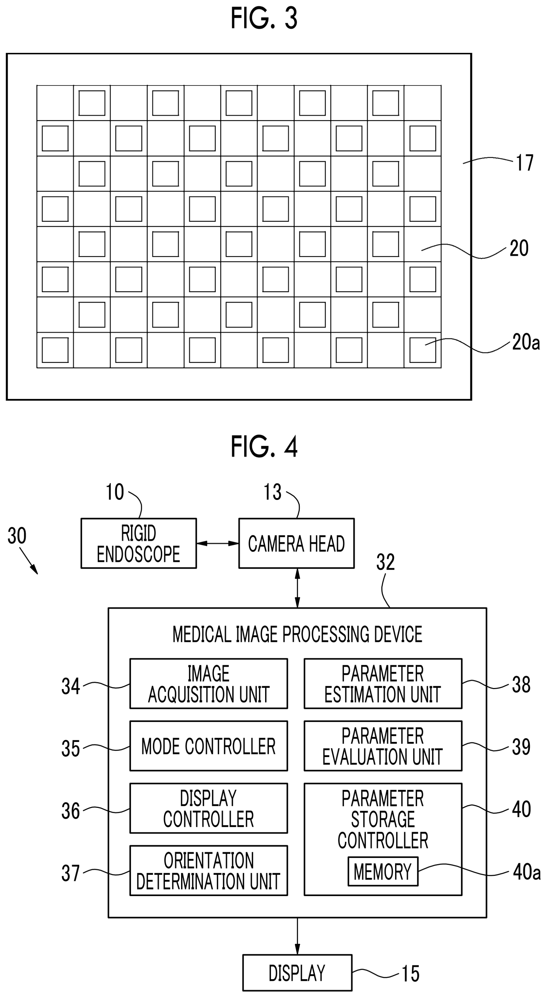

In a case where the rigid endoscope 10 is newly mounted on the camera head 13 or in a case where another rigid endoscope 10 is mounted, calibration is performed. The calibration is performed by switching to a calibration mode and then imaging a calibration plate 17 with a distal end part of the rigid endoscope 10 facing the calibration plate 17 as shown in FIG. 2. As shown in FIG. 3, the calibration plate 17 is provided with a pattern 20 used for correcting the image. The pattern 20 has a lattice shape, and is used to correct distortion in the image caused by using a wide-angle optical system in the rigid endoscope 10 due to lattice distortion. The switching to the calibration mode is automatically performed in a case where the pattern 20 is recognized in the image. The automatic switching to the calibration mode will be described in detail below. It is preferable that a marker such as an AR marker be provided on a double frame lattice 20a having double frames in the pattern 20. In addition, the pattern 20 is not limited to a regular shape such as a lattice pattern, and may have an irregular shape.

The endoscope system 30 comprises a medical image processing device 32 that processes the image obtained from the camera head 13. The image processed by the medical image processing device 32 is displayed on the display 15. The medical image processing device 32 stores programs for performing various types of processing in a program memory (not shown). A central controller (not shown) configured by a processor executes the programs in the program memory, thereby realizing functions of an image acquisition unit 34, a mode controller 35, a display controller 36, an orientation determination unit 37, a parameter estimation unit 38, a parameter evaluation unit 39, and a parameter storage controller 40, as shown in FIG. 4. The endoscope system 30 may comprise a light source device that supplies illumination light to the rigid endoscope 10.

In addition to processing the image obtained by the rigid endoscope 10, the medical image processing device 32 may also process an endoscope image obtained by a gastrointestinal endoscope, and may also perform processing such as extracting and analyzing a three-dimensional image from a tomographic image such as a computed tomography (CT) image or a magnetic resonance imaging (MRI) image. The medical image processing device 32 may be a part of a surgical robot or may be a console that controls the surgical robot. In addition, in the present embodiment, the calibration in the endoscope system 30 in which a doctor operates the rigid endoscope 10 has been described, but the present disclosure is not limited to this. The present disclosure can also be applied to calibration in a system in which a surgical robot operates an operating member for the patient P, in which an existing rigid endoscope or the like (a rigid endoscope that can be used for purposes other than a robot) is used in combination as the operating member.

The image acquisition unit 34 acquires an image. The image is acquired from the rigid endoscope 10 via the camera head 13. The image is acquired as a sequence of video images for each frame. The image to be acquired includes an image obtained before the rigid endoscope 10 is inserted into the patient, as well as an image of the abdominal cavity obtained by the rigid endoscope 10 inserted into the patient. In the image of the abdominal cavity, in addition to the organ of the patient, the forceps 11, an ultrasound probe, and the like are shown.

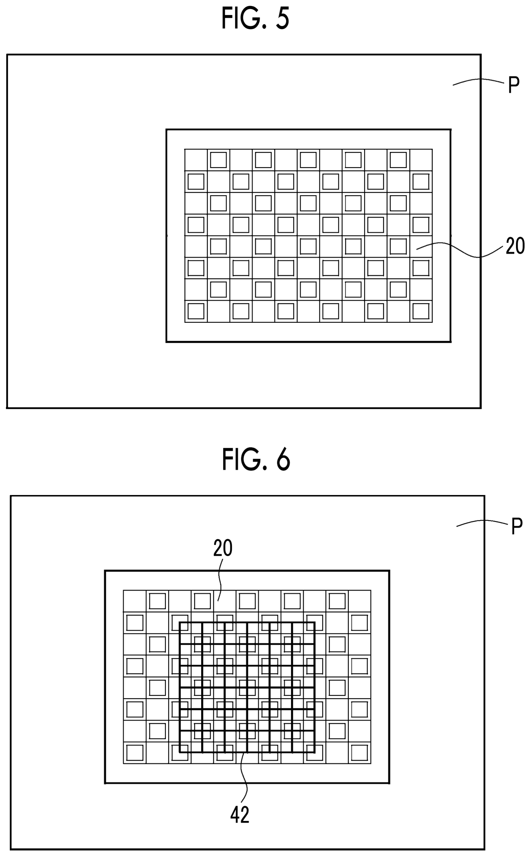

The mode controller 35 switches to the calibration mode in a case where the mode controller 35 recognizes, from the image, a pattern used for correcting the image. In the calibration mode, the pattern 20 provided on the calibration plate 17 is used, as described above. As shown in FIG. 5, in a case where the pattern 20 is recognized from an image P, the mode controller 35 automatically switches to the calibration mode, thereby eliminating the need for the doctor to go through the trouble of switching to the calibration mode. It is preferable that the pattern recognition be performed not only by a pattern-matching process with the pattern 20 stored in advance, but also by using a learning model that has learned the pattern 20 through machine learning.

It is preferable that the mode controller 35 switch the calibration mode to a normal mode in a case where a release condition related to the correction is satisfied, and maintain the calibration mode in a case where the release condition is not satisfied. In a case where the calibration mode is cancelled, the mode controller 35 can automatically switch the mode, as in a case where the calibration mode is started, thereby eliminating the need for the doctor to go through the trouble. It is preferable that the release condition be that an evaluation of the correction is appropriate. It is preferable that the evaluation of the correction be performed by the parameter evaluation unit 39 described below. It is preferable that the normal mode be a mode for observing the abdominal cavity of the patient.

The display controller 36 displays a guide pattern for guiding the pattern on the image in the calibration mode. Specifically, as shown in FIG. 6, on the image P of the display 15, a guide pattern 42 is displayed on the pattern 20. The guide pattern 42 has a lattice shape, and an angle of view of the rigid endoscope 10 is adjusted by moving the rigid endoscope 10 such that the lattice of the guide pattern 42 and the lattice of the pattern 20 overlap each other.

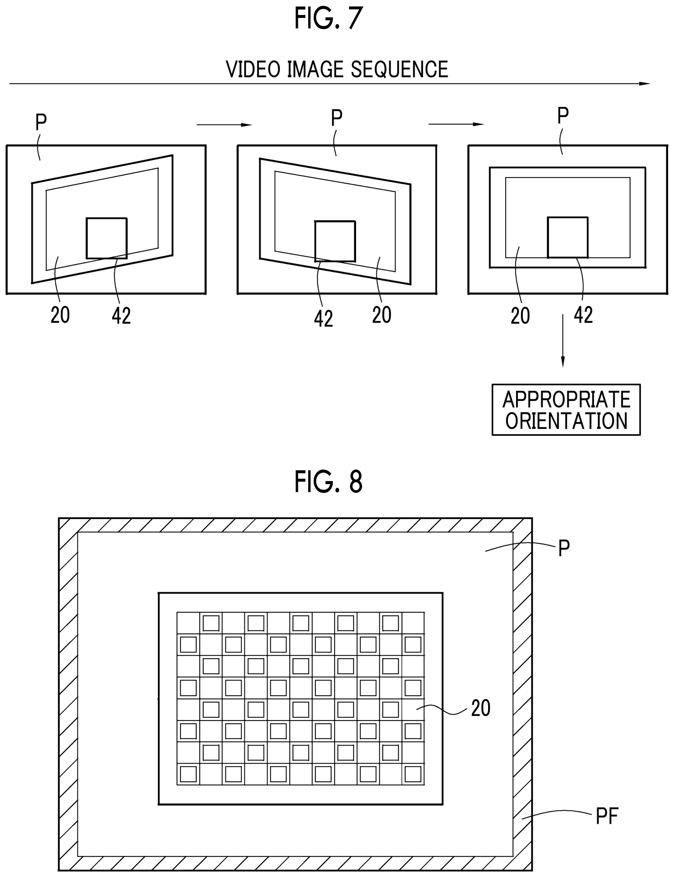

The orientation determination unit 37 determines whether or not an orientation of the pattern 20 on the image is appropriate as an orientation for performing the correction. Specifically, as shown in FIG. 7, in a sequence of video images, the orientation determination unit 37 determines whether or not the lattice of the guide pattern 42 and the lattice of the pattern 20 overlap each other. In a case where the orientation determination unit 37 determines that the lattice of the guide pattern 42 and the lattice of the pattern 20 overlap each other, the display controller 36 notifies that the orientation of the pattern 20 on the image is appropriate. As a method of the notification, for example, as shown in FIG. 8, it is preferable to change a color of an outer frame PF of the image (a change in color of the outer frame PF is represented by hatching).

In order to select an appropriate image for estimating a camera parameter, the orientation determination unit 37 may evaluate the number of markers (for example, AR markers) provided on the pattern 20, a normal direction of a calibration tool, or the like to determine whether or not the orientation of the pattern 20 is appropriate. In a case where a marker is provided on the pattern 20, the orientation determination unit 37 may calculate an index indicating whether the marker faces the rigid endoscope 10, and determine whether or not the orientation of the pattern 20 is appropriate based on the calculated index. In addition, the orientation determination unit 37 may determine whether or not the orientation of the pattern 20 is appropriate based on a distance from the pattern 20 in addition to the orientation of the pattern 20.

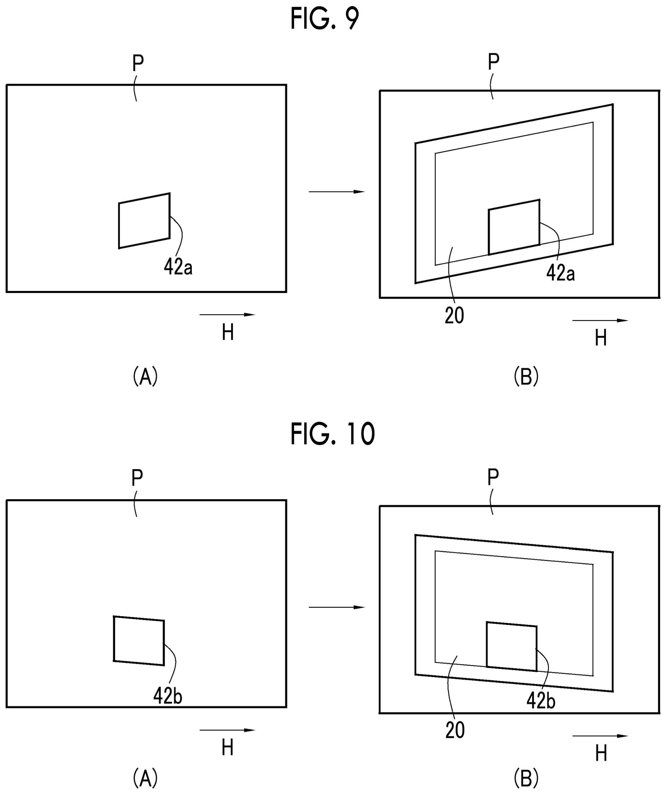

As an image in which the orientation of the pattern 20 is appropriate, a plurality of images in which the pattern 20 has different orientations, such as a tilt or a direction, may be acquired. In this case, it is preferable that the display controller 36 change the display of the guide pattern in accordance with the orientation of the pattern that is deemed appropriate as the orientation for performing the correction. For example, in a case where the orientation of the pattern 20 tilted upward with respect to a horizontal direction H is deemed to be an appropriate orientation, a guide pattern 42a for a first orientation tilted upward with respect to the horizontal direction H is displayed, as shown in (A) of FIG. 9. In this case, as shown in (B) of FIG. 9, in a case where the orientation determination unit 37 determines that the lattice of the guide pattern 42a and the lattice of the pattern 20 overlap each other, the image for the first orientation is stored in a predetermined memory (not shown).

Next, in a case where the orientation of the pattern 20 tilted downward with respect to the horizontal direction H is deemed to be an appropriate orientation, the display is switched from the guide pattern 42a for the first orientation to a guide pattern 42b for a second orientation tilted with respect to the horizontal direction H, as shown in (A) of FIG. 10. As shown in (B) of FIG. 10, in a case where the orientation determination unit 37 determines that the lattice of the guide pattern 42b and the lattice of the pattern 20 overlap each other, the image for the second orientation is stored in the predetermined memory. As described above, by using the two images for the first and second orientations stored in the predetermined memory for correcting the image, the accuracy of the correction can be improved.

The parameter estimation unit 38 estimates parameters of the correction from feature amounts of the image. Specifically, as shown in FIG. 11, from the image in which it is determined that the lattice of the guide pattern 42 and the lattice of the pattern 20 overlap each other, three-dimensional coordinates of feature points are calculated, and correction parameters are estimated from the three-dimensional coordinates of the feature points. It is preferable that a known method be used as a method of estimating the parameter.

The parameter estimation unit 38 may estimate the parameters of the correction from a plurality of images in which the pattern has different orientations. In this case, it is preferable to acquire a plurality of images in which the pattern 20 has an appropriate orientation for correction, as the plurality of images in which the pattern has different orientations. For example, the image of the pattern 20 having the orientation tilted upward with respect to the horizontal direction H and the image of the pattern 20 having the orientation tilted downward with respect to the horizontal direction H are acquired, and the parameters are estimated based on the images of the patterns having the two orientations (see FIGS. 9 and 10).

The parameter evaluation unit 39 evaluates the parameters based on the image reflecting the parameters. Specifically, as shown in FIG. 12, for an image acquired after the parameter estimation, an image appropriate for evaluation is acquired from images obtained by imaging the pattern 20 in a sequence of video images. The image appropriate for evaluation is the same as the image appropriate for correction, and it is preferable that the orientation of the pattern included in the image appropriate for evaluation be the same as the orientation of the pattern included in the image used for the parameter estimation. In a case where the image appropriate for evaluation is acquired, the parameter evaluation unit 39 reflects the parameters in the image. Then, feature points are extracted from a corrected image after the parameters are reflected, and a curvature of a line connecting each feature point is calculated as an index for evaluation. The evaluation is performed based on the calculated index. The result of the evaluation is transmitted to the mode controller 35 and used for controlling the mode.

In a case where the evaluation is appropriate, the parameter storage controller 40 stores the parameters in a memory 40a. The parameters stored in the memory 40a are used for correcting the distortion of the image. As a result, for example, even in a case where an ultrasound image is superimposed on a rigid endoscope image, the ultrasound image can be superimposed without misregistration, and the orientation of the organ can also be accurately detected. Therefore, a 3D model can be accurately reconstructed in accordance with the state of the orientation. As for the pattern used for evaluation, in addition to the parameter estimation pattern 20, an evaluation pattern 22 different from that for parameter estimation may be used as shown in FIG. 13.

Next, a series of flows of the calibration will be described with reference to a flowchart of FIG. 14. The doctor D performs a laparoscopic surgery while viewing the image obtained by the rigid endoscope 10 displayed on the display 15. In a case of replacing the rigid endoscope 10 with another one, the doctor pulls out the rigid endoscope 10 from a port inserted into the patient. The doctor D takes the rigid endoscope 10 that is about to be inserted into the patient, and images the calibration plate 17 with the taken rigid endoscope 10. The image is obtained by imaging using the rigid endoscope 10. The image acquisition unit 34 acquires the image P. The image P is displayed on a screen of the display 15.

The mode controller 35 switches to the calibration mode in a case where the mode controller 35 recognizes, from the image P, the pattern 20 used for correcting the image P. In the calibration mode, the display controller 36 displays, on the image P, the guide pattern 42 for guiding the pattern 20. The doctor moves the rigid endoscope 10 or the calibration plate 17 such that the pattern 20 on the image and the guide pattern 42 overlap each other. The orientation determination unit 37 determines whether or not an orientation of the pattern 20 on the image is appropriate as an orientation for performing the correction.

Then, in a case where the pattern 20 on the image and the guide pattern 42 overlap each other and the orientation determination unit 37 determines that the orientation of the pattern 20 is appropriate, an image at the time of the determination is acquired as the image used for the parameter estimation. The parameter estimation unit 38 estimates parameters of the correction from feature points of the image. The parameter evaluation unit 39 evaluates the correction based on the image reflecting the parameters. In a case where the evaluation is appropriate, the parameter storage controller 40 stores the parameters in the memory 40a. Since the evaluation is appropriate, the release condition related to the correction is satisfied, and thus the mode controller 35 switches from the calibration mode to the normal mode. Then, the doctor D inserts the rigid endoscope 10 into the port and resumes the surgery. As a result, the calibration is completed.

In the present embodiment, each processing of the image acquisition unit 34, the mode controller 35, the display controller 36, the orientation determination unit 37, the parameter estimation unit 38, the parameter evaluation unit 39, and the like is executed by any computer. In addition, any computer may execute the processing using a processor, a program, or a combination thereof. Any computer may be a general-purpose computer, a computer for a specific use, a system such as a workstation, or other hardware elements capable of executing a program.

The processor may be configured by one or more pieces of hardware, and the type of hardware is not limited. For example, the processor may be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphics processing unit (GPU) or a neural processing unit (NPU). In addition, the processor has each unit or each means that executes various types of processing in the present embodiment. In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each processing executed by the processor is not limited to the above order and may be changed as appropriate. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a recording medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Explanation of References

10: rigid endoscope
11: forceps
13: camera head
15: display
17: calibration plate
20, 22: pattern
20a: double frame lattice
30: endoscope system
32: medical image processing device
34: image acquisition unit
35: mode controller
36: display controller
37: orientation determination unit
38: parameter estimation unit
39: parameter evaluation unit
40: parameter storage controller
40a: memory
42, 42a, 42b: guide pattern
T: surgical target part
D: doctor
C: cover
CO: opening portion
P: image
PF: outer frame
H: horizontal direction

## Claims

1. A medical image processing device (32) comprising:
a processor,
wherein the processor is configured to
acquire an image, and
switch to a calibration mode in a case where a pattern (20, 22) used for correcting the image is recognized from the image.

2. The medical image processing device (32) according to claim 1,
wherein the processor is configured to switch from the calibration mode to a normal mode in a case where a release condition related to the correction is satisfied, and maintain the calibration mode in a case where the release condition is not satisfied.

3. The medical image processing device (32) according to claim 2,
wherein the release condition is that an evaluation of the correction is appropriate.

4. The medical image processing device (32) according to claim 2,
wherein the processor is configured to estimate a parameter of the correction from feature points of the image.

5. The medical image processing device (32) according to claim 4,
wherein the processor is configured to estimate the parameter from a plurality of the images in which the pattern (20, 22) has different orientations.

6. The medical image processing device (32) according to claim 4,
wherein the processor is configured to evaluate the correction based on the image to which the parameter has been applied.

7. The medical image processing device (32) according to claim 6,
wherein the processor is configured to store the parameter in a case where the evaluation is appropriate.

8. The medical image processing device (32) according to claim 1,
wherein the processor is configured to display a guide pattern (42, 42a, 42b) for guiding the pattern (20, 22) on the image in the calibration mode.

9. The medical image processing device (32) according to claim 8,
wherein the processor is configured to change the display of the guide pattern (42, 42a, 42b) in accordance with an orientation of the pattern (20, 22) that is deemed appropriate as an orientation for performing the correction.

10. The medical image processing device (32) according to claim 1,
wherein the processor is configured to
determine whether or not an orientation of the pattern (20, 22) on the image is appropriate as an orientation for performing the correction, and
in a case where the orientation of the pattern (20, 22) is appropriate, notify that the orientation of the pattern (20, 22) is appropriate.

11. A method of operating a medical image processing device (32) including a processor, the method comprising:
a step of acquiring an image via the processor; and
a step of switching to a calibration mode in a case where the processor recognizes, from the image, a pattern (20, 22) used for correcting the image.
